# EUROPÄISCHE PATENTSCHRIFT

(11) **EP 2 560 605 B1**
(45) Veröffentlichungstag und Bekanntmachung des Hinweises auf die Patenterteilung: **27.02.2019**
(21) Anmeldenummer: 11712503.9
(22) Anmeldetag: 23.03.2011
(51) Int. Cl.: A61K 36/575, A61K 8/06, A61K 8/34, A61K 8/86, A61K 8/39, A61K 8/46, A61K 8/97, A61K 9/00, A61K 9/06, A61K 9/10, A61K 47/26, A61K 47/34, A61Q 19/08

(54) **WIRKSTOFFKOMBINATIONEN AUS MAGNOLIENRINDENEXTRAKT UND POLYETHOXYLIERTEN VERBINDUNGEN (PEG-40-STEARATE)**
ACTIVE AGENT COMBINATIONS COMPRISING MAGNOLIA BARK EXTRACT AND POLYETHOXYLATED COMPOUNDS (PEG-40-STEARATE)
COMBINAISONS DE PRINCIPES ACTIFS COMPRENANT UN EXTRAIT D' ECORCE DE MAGNOLIA ET DES AGENTS TENSIOACTIFS POLYETHOXYLÉS (PEG-40-STEARATE)

(30) Priorität: 20.04.2010 DE 102010015791
(43) Veröffentlichungstag der Anmeldung: 27.02.2013
(73) Patentinhaber: Beiersdorf AG, 20253 Hamburg (DE)
(72) Erfinder: SCHLÄGER, Torsten, 22297 Hamburg (DE); ECKERT, Julia, 20249 Hamburg (DE); NEUFANG, Gitta, 20149 Hamburg (DE); PETERS, Nils, 22965 Todendorf (DE); KNAUPMEIER, Stefanie, 32108 Bad Salzuflen (DE); HEUSER, Stefan, 90762 Fürth (DE)
(86) Internationale Anmeldenummer: PCT/EP2011/054453
(87) Internationale Veröffentlichungsnummer: WO 2011/131445

(56) Entgegenhaltungen:
- WO-A1-03/030858
- WO-A1-2007/064519
- CN-A- 101 653 518
- FR-A1- 2 911 507
- KR-A- 20090 002 371
- US-A1- 2008 260 869

## Beschreibung

Die vorliegende Erfindung betrifft kosmetische oder dermatologische Zubereitungen zur Behandlung, Pflege und Prophylaxe von sensibler Haut und erythematösen Erscheinungen.

Das Problem "sensible Haut" betrifft eine wachsende Anzahl Erwachsener und Kinder. Mit sensibler Haut bezeichnet man eine Kombination verschiedener Symptome, wie hyperreaktive und intolerante Haut. Aber auch atopische Haut kann darunter subsummiert werden. Diese Hautzustände werden von den Betroffenen oftmals, nicht ganz korrekt, als "allergische" Haut bezeichnet. Obwohl eine allergische Erkrankung zu Symptomen der sensiblen Haut führen kann, ist die Erscheinung "sensible Haut" nicht auf Allergiker beschränkt.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

Bei atopischer Haut ist der Juckreiz als neurosensorisches Phänomen anzusehen.

"Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluß wie Sonne, Kälte, Trockenheit, aber auch feuchte Wärme, Wärmestrahlung und UV-Strahlung, z.B. der Sonne, hervorgerufen werden.

Nach bisherigen Erkenntnissen tritt eine derartige Empfindlichkeit gegenüber ganz bestimmten Substanzen individuell unterschiedlich auf. Dies bedeutet, eine Person, die bei Kontakt mit einer Substanz "Stingingeffekte" erlebt, wird sie mit hoher Wahrscheinlichkeit bei jedem weiteren Kontakt wiederholt erleben. Der Kontakt mit anderen "Stingern" kann aber ebensogut ohne jede Reaktion verlaufen.

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Daher sollten insbesondere Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, ins-besondere Dermatosen, aber auch des Erscheinungsbildes des "Stingings" zur Verfügung gestellt werden, die solche erythematösen Hauterscheinungen lindern.

Erfindungsgemäß werden diese Aufgaben gelöst durch die Verwendung von Magnolienrindenextrakt zur Herstellung von kosmetischen oder dermatologischen Zubereitungen zur Behandlung, Pflege und Prophylaxe von sensibler Haut und/oder zur Behandlung und Prophylaxe von erythematösen Erscheinungen.

Magnolienrindenextrakt ist bekanntermaßen ein antimikrobiell wirksames Agens und wurde seit langem in der indianischen Volksmedizin verwendet. Nachteilig ist, daß solche Extrakte leichtverderblich sind, und sich bereits in kurzer Frist durch Lichteinfall, Luftzutritt und Temperatureinflüsse dunkel zu verfärben beginnen, wodurch ihre Wirksamkeit meßbar abnimmt.

Eine weitere Aufgabe der vorliegenden Erfindung bestand daher darin, Wirkstoffe bzw. Zubereitungen, solche Wirkstoffe enthaltend, zur Verfügung zu stellen, die sich durch eine erhöhte Beständigkeit gegenüber Licht- und/oder Lufteinwirkung und/oder Temperatureinflüssen auszeichnen würden.

Es wurde überraschend gefunden, und darin liegt die Lösung all dieser Aufgaben, daß Wirkstoffkombinationen aus
a) einem mit Hilfe von superkritischem CO₂ oder Ethanol oder einer Ethanol / Wasser Mischung gewonnenem Magnolienrindenextrakt mit einem Gehalt an Magnolol und/oder Honokiol
   und
b) und einer oder mehreren ober- oder grenzflächenaktiven polyethoxylierten Verbindungen,
dadurch gekennzeichnet, dass die ober- oder grenzflächenaktiven polyethoxylierten Verbindung oder Verbindungen gewählt wird oder werden aus der Gruppe Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(30)stearat, Polyethylenglycol(40)stearat, Polyethylenglycol(100)stearat, Polyethylenglycol(200)stearat, Polyethylenglycol-(400)stearat, Polyethylenglycol(2)stearylether, Polyethylenglycol(21)stearylether, Polyethylenglycol(20)cetylstearylether, Polyethylenglycol(40) hydriertes Rizinusöl, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat
den Nachteilen des Standes der Technik abhelfen.

Schließlich besteht eine vorteilhafte Ausführungsform der vorliegenden Erfindung in der Verwendung
b) einer oder mehreren ober- oder grenzflächenaktiven polyethoxylierten Verbindungen zur Stabilisierung eines
a) mit Hilfe von superkritischem CO2 oder Ethanol oder einer Ethanol / Wasser Mischung gewonnenen Magnolienrindenextraktes mit einem Gehalt an Magnolol und/oder Honokiol
gegen den Verderb durch Licht- und/oder Lufteinwirkung und/oder Temperatureinflüsse,
dadurch gekennzeichnet, dass die ober- oder grenzflächenaktiven polyethoxylierten Verbindung oder Verbindungen gewählt wird oder werden aus der Gruppe Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(30)stearat, Polyethylenglycol(40)stearat, Polyethylenglycol(100)stearat, Polyethylenglycol(200)stearat, Polyethylenglycol-(400)stearat, Polyethylenglycol(2)stearylether, Polyethylenglycol(21)stearylether, Polyethylenglycol(20)cetylstearylether, Polyethylenglycol(40) hydriertes Rizinusöl, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

Bei Anwendung der erfindungsgemäßen Wirkstoffkombinationen ist eine wirksame Behandlung, aber auch eine Prophylaxe von entzündlichen Hautzuständen - auch dem atopischen Ekzem - möglich. Kosmetische oder topische dermatologische Zubereitungen mit einem wirksamen Gehalt an den erfindungsgemäßen Wirkstoffkombinationen dienen aber auch in überrachender Weise zur Beruhigung von empfindlicher oder gereizter Haut.

Die WO2007064519 A und die CN101653518 A konnten nicht den Weg zur vorliegenden Erfindung weisen.

Magnolien (Magnolia) sind eine Pflanzengattung in der Familie der Magnoliengewächse (Magnoliaceae). Diese enthält etwa 230 Arten, die aus Ostasien und Amerika stammen. Erfindungsgemäß kann der Magnolienrindenextrakt grundsätzlich allen Arten der Gattung Magnolia entnommen werden, wobei allerdings Magnolia grandiflora, und insbesondere Magnolia officinalis bevorzugt werden.

Magnolien-Rinde wird in der Traditionellen Chinesischen Medizin (TCM) vor allem bei "Stagnation of qi" (low energy), emotionalem Stress, Entzündungen, innerer Unruhe und Angstzuständen eingesetzt.

Die Herstellung des verwendeten Extraktes erfolgt mit Hilfe von superkritischem CO₂ oder Ethanol oder einer Ethanol / Wasser Mischung.

Als Wirkkomponenten und Leitsubstanzen des Extraktes sind Magnolol und Honokiol beschrieben.

Magnolol ist durch folgende Struktur gekennzeichnet:

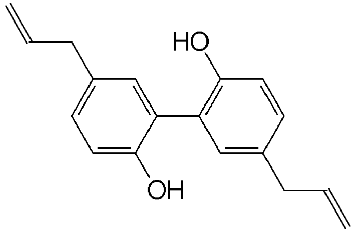

Honokiol ist durch folgende Struktur gekennzeichnet:

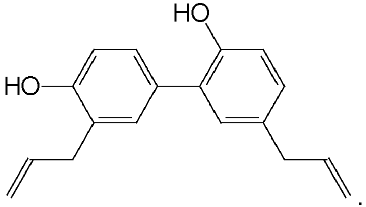

Erfindungsgemäß werden die Wirkstoffkombinationen bevorzugt in kosmetischen oder dermatologischen Zusammensetzungen eingesetzt mit einem Gehalt von 0,005 - 50,0 Gew.-%, insbesondere 0,01 - 20,0 Gew.-%, bezogen auf das Gesamtgewicht der Zusammensetzung. Vorteilhaft enthalten die Zusammensetzungen 0,02 - 10,0 Gew.-%, besonders bevorzugt 0,02 - 5,0 Gew.-% an den Wirkstoffkombinationen, ganz besonders vorteilhaft 0,05 - 3,0 Gew.-%, jeweils bezogen auf das Gesamtgewicht der Zusammensetzung.

Erfindungsgemäße, vorteilhaft als Emulsionen vorliegende Zubereitungen enthalten eine oder mehrere ober- oder grenzflächenaktiven polyethoxylierten Verbindungen, die beispielsweise vorteilhaft gewählt werden können aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten ober- oder grenzflächenaktiven polyethoxylierten Verbindungen gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(2)stearylether (Steareth-2), Polyethylenglycol(14)stearylether (Steareth-14), Polyethylenglycol(16)stearylether (Steareth-16), Polyethylenglycol(17)stearylether (Steareth-17), Polyethylenglycol(18)stearylether (Steareth-18), Polyethylenglycol-(19)stearylether (Steareth-19), Polyethylenglycol(20)stearylether (Steareth-20), Polyethylenglycol(21)stearylether (Steareth-21),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19-), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),
Es ist ferner von Vorteil, die Fettsäureethoxylate aus folgender Gruppe zu wählen:
   Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(30)stearat, Polyethylenglycol(40)stearat, Polyethylenglycol(100)stearat, Polyethylenglycol(200)stearat, Polyethylenglycol(400)-stearat.

Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat
Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Ebenfalls vorteilhaft sind ethoxylierte Rizinusölverbindungen wie Polyethylenglycol(40) hydriertes Rizinusöl (INCI: Polyethylenglycol(40) hydrogenated Castor oil).

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Bevorzugte ober- oder grenzflächenaktive polyethoxylierten Verbindungen im Sinne der vorliegenden Erfindung sind Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(30)stearat, Polyethylenglycol(40)stearat, Polyethylenglycol(100)stearat, Polyethylenglycol(200)stearat, Polyethylenglycol(400)stearat, Polyethylenglycol(2)stearylether, Polyethylenglycol-(21)stearylether, Polyethylenglycol(20)cetylstearylether, Polyethylenglycol(40) hydriertes Rizinusöl, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

Die erfindungsgemäß verwendeten Wirkstoffkombinationen lassen sich ohne Schwierigkeiten in gängige kosmetische oder dermatologische Formulierungen einarbeiten, vorteilhaft in Pumpsprays, Aerosolsprays, Cremes, Salben, Tinkturen, Lotionen, Nagelpflegeprodukte (z.B. Nagellacke, Nagellackentferner, Nagelbalsame) und dergleichen.

Es ist auch gegebenenfalls vorteilhaft, die Zusammensetzungen gemäß der Erfindung abzupuffern. Vorteilhaft ist ein pH-Bereich von 3,5 - 7,5. Besonders günstig ist es, den pH-Wert in einem Bereich von 4,0 - 6,5 zu wählen.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetischen und dermatologischen Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdikkungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, mindestens eine dialkylsubstituierte Carbonsäure im wäßrigen Medium und Hilfsmittel, wie sie üblicherweise dafür verwendet werden.

### Wirkungsnachweis

Magnolienrindenextrakt enthaltend Magnolol und / oder Honokiol weist bei Lagerung im Licht sowie bei höheren Temperaturen (Brutschrank 40°C) eine Instabilität auf. Diese Instabilität ist vermutlich auf eine Zersetzung der beiden aktiven Komponenten Magnolol und Honokiol zurückzuführen und äußert sich in einer Verfärbung des Materials.

Auch in kosmetischen Formulierungen kann diese Instabilität normalerweise nicht verhindert werden. Auch hier wird nach längerer Lichtexposition bzw. Lagerung bei erhöhter Temperatur (40°C) eine Verfärbung des Ansatzes beobachtet.

Durch die Verwendung eines erfindungsgemäß ausgewählten O/W-Emulgators (PEG-40-Stearat) konnte überraschenderweise eine Stabilisierung von Magnolienrindenextrakt in kosmetischen Formulierungen gezeigt werden. Sowohl nach Lichtexposition als auch nach Lagerung bei erhöhter Temperatur (40°C) kam es zu keiner Verfärbung der Ansätze.

### Beschreibung der Erfindung und der durchgeführten Versuche

Es wurden jeweils 3 Ansätze mit sechs verschiedenen Emulgatoren durchgeführt, bei denen jeweils der Emulgator alleine, der Magnolienrindenextrakt alleine und eine Mischung aus Emulgator und Magnolienrindenextrakt in Wasser und Butylenglykol gelöst wurden.

Als Emulgatoren wurden PEG-40-Stearat, Na-Stearoyl Glutamat und Na-Cetearylsulfat (Mischung aus Na-Cetylsulfat und Na-Stearylsulfat) verwendet.

Die Einsatzmengen sowie die Versuchsbedingungen waren bei allen Ansätzen identisch (2g Emulgator, 0,5g Magnolienrindenextrakt, 10g Butylenglykol, Wasser ad 100g).

Nach zwei Monaten Lagerung im Licht und bei erhöhter Temperatur (40°) wurde die Färbung der Ansätze durch ein Expert Grading mittels einer Farbschablone bewertet.

### Ergebnisse:

Die Auswertung mittels Farbschablone wurde folgendermaßen vorgenommen:
Anhand verschiedener Farbtafeln wurde zunächst die Farbe zu t₀ bestimmt. Die Farbveränderung nach t_{2 Monate} wird durch Δ x% angegeben.

**Bewertung der Färbung der B40-Ansätze durch Expert Grading mittels Farbschablone**

| | Magnolienrindenextrakt + Butylenglykol + H₂O | Emulgator + Magnolienrindenextrakt + Butylenglykol + H₂O |
|---|---|---|
| | t_{2Monate} | t_{2Monate} |
| PEG-40-Stearat | Δ = 60% | Δ = 10% |
| Natrium-Stearoylglutamat | Δ = 70% | Δ = 80% |
| Natrium-Cetearylsulfat | Δ = 60% | Δ = 80% |

Bei Verwendung von PEG-40-Stearat konnte eine Stabilisierung von Magnolienrindenextrakt in kosmetischen Formulierungen gezeigt werden. Sowohl nach Lichtexposition als auch nach Lagerung bei erhöhter Temperatur (40 °C) kam es zu keiner Verfärbung der Ansätze.

Bei Verwendung der Emulgatoren Natriumcetearylsulfat und Natriumstearoylglutamat indessen war keine nennenswerte Stabilisierung zu beobachten.

Die folgenden Beispiele sollen die Verkörperungen der vorliegenden Erfindungen verdeutlichen. Die Angaben beziehen sich stets auf Gewichts-%, sofern nicht andere Angaben gemacht werden.

**Creme (Beispiel 1 bis 3)**

| | **Nr. 1** | **Nr. 2** | **Nr. 3** |
|---|---|---|---|
| PEG-40 Stearat | 1,00 | 1,00 | 1,00 |
| Glycerylstearat | 3,00 | 3,00 | 3,00 |
| C12-15 Alkylbenzoat | 2,50 | 2,00 | 2,50 |
| Caprylsäure/Caprinsäuretriglyceride | 2,50 | 3,00 | 2,50 |
| Cetearylalkohol | 3,00 | 3,00 | 3,00 |
| Octyldodecanol | 3,00 | 3,00 | - |
| Cyclomethicon | 2,00 | 2,00 | 2,00 |
| Dicaprylylcarbonat | 2,50 | 2,50 | 2,50 |
| Dimethicon | 0,50 | 1,00 | 1,00 |
| Glycerin | 7,50 | 4,00 | 8,60 |
| Methylparaben | 0,20 | 0,20 | 0,20 |
| Phenoxyethanol | 0,40 | 0,40 | 0,40 |
| Propylparaben | 0,10 | - | 0,10 |
| Natrium metabisulfit | - | 0,10 | 0,10 |
| Butylenglykol + lodpropinylbutylcarbamat | - | 0,10 | - |
| Carbomer | 0,30 | 0,30 | 0,10 |
| Natrium polyacrylat | 0,40 | - | 0,40 |
| Trisnatrium EDTA | - | 1,00 | - |
| BHT | - | 0,05 | - |
| Butyrospermum Parkii Butter | - | - | 2,00 |
| Talk | 1,00 | - | - |
| Aluminium Stärke Octenylsuccinat | - | 1,00 | - |
| Butylenglykol | - | 4,00 | 3,00 |
| Magnolienrindenextrakt | 0,10 | 0,5 | 2,00 |
| Natriumhydroxid | q.s. | q.s. | q.s. |
| Parfum | q.s. | q.s. | q.s. |
| Ethylhexylmethoxycinnamat | 3,00 | - | - |
| Butylmethoxydibenzoylmethan | 2,00 | - | - |
| Octocrylene | - | 1,0 | - |
| Phenylbenzimidazol sulfonsäure | - | - | 1,0 |
| Wasser | ad 100 | ad 100 | ad 100 |

**Creme (Beispiel 4 und 5)**

| | **Nr. 4** | **Nr. 5** |
|---|---|---|
| Polyethylenglykol(21)stearylether | 2,00 | 1,50 |
| Polyethylenglykol(2)stearylether | 2,50 | 2,50 |
| Polypropylenglykol(15)stearylether | 3,00 | 4,00 |
| Octyldodecanol | 2,00 | - |
| Dicaprylylether | 0,50 | 1,50 |
| Na₃HEDTA (20% wäßr. Lösung) | 1,50 | 1,50 |
| Avocadoöl | 0,10 | 0,10 |
| Parfum, Antioxidantien | q.s. | q.s. |
| Magnolienrindenextrakt | 0,15 | 0,3 |
| Wasser | ad 100 | ad 100 |

**Creme (Beispiel 6 und 7)**

| | **Nr. 6** | **Nr. 7** |
|---|---|---|
| Ceteareth-20 | 1,5 | 2,5 |
| Glycerylstearate + Ceteareth-20 + Cetylstearylalkohol + Cetylpalmitat + Ceteareth-12 | 3 | 4 |
| Cera Microcristallina + Paraffinum Liquidum | - | 1 |
| Paraffinum Liquidum | 2 | 1 |
| Butyrospermum Parkii Butter | - | 0,5 |
| Dicaprylylcarbonat | 1 | 1 |
| Butylenglycoldicaprylate/Dicaprat | 1 | 1 |
| C12-15 Alkylbenzoat | 4 | 2 |
| Butylmethoxydibenzoylmethan | 0,5 | 1 |
| 4-Methylbenzylidencampher | - | 1 |
| Dinatriumphenyldibenzimidazoltetrasulfonat | 0,5 | - |
| Aloe Barbadensis Gel | - | 0,1 |
| Panthenol | - | 0,2 |
| Magnolienrindenextrakt | 0,5 | 1,0 |
| Phenoxyethanol | 0,3 | 0,4 |
| Methylparaben | 0,1 | 0,1 |
| Alkohol, Denat. | 1 | 1 |
| Glycerin | 3 | 5 |
| Tocopheryl Acetat | 0,1 | - |
| Methylpropanediol | 1 | - |
| Natriumhydroxid | q.s. | q.s |
| Parfum | q.s. | q.s |
| Citronensäure | q.s. | q.s. |
| Wasser | ad 100 | ad 100 |

**Creme (Beispiel 8 und 9)**

| | **Nr. 8** | **Nr. 9** |
|---|---|---|
| Polyoxyethylen(20)cetylstearylether | 3,0 | 4,0 |
| Polyoxyethylen(12)cetylstearylether | 0,5 | - |
| Glycerinstearat | 3,0 | 3,0 |
| Cetylstearylalkohol | 0,5 | - |
| Cetylpalmitat | 0,5 | - |
| Caprylsäure/Caprinsäuretriglyceride | 2,0 | 3,0 |
| Octyldodecanol | 3,0 | 2,0 |
| Polyethylenglycol(150)distearat | - | 1,0 |
| Dicaprylyl ether | 1,0 | 1,0 |
| Glycerin | 4,0 | 2,0 |
| Magnolienrindenextrakt | 0,15 | 0,3 |
| Parfum, Antioxidantien | q.s. | q.s. |
| Wasser | ad 100 | ad 100,00 |

**Creme (Beispiel 10 und 11)**

| | **Nr. 10** | **Nr. 11** |
|---|---|---|
| Palmitinsäure | 1,50 | - |
| Stearylalkohol | 3,00 | - |
| Cetylalkohol | - | 3,00 |
| PEG-100 Stearat | 3,50 | 4,00 |
| C12-15 Alkybenzoat | 5,00 | 5,00 |
| Octyldodecanol | - | 2,00 |
| Dicaprylylether | 2,00 | - |
| Cyclomethicon | 3,00 | 1,00 |
| Methylpropandiol | - | 4,00 |
| Propylenglykol | 5,00 | - |
| Glycerin | 4,00 | 2,00 |
| Diethylaminohydroxybenzoylhexylbenzoat | 1,00 | - |
| Dinatriumphenyldibenzimidazoltetrasulfonat | - | 2,00 |
| Phenylbenzimidazolsulfonsäure | - | 3,00 |
| Ethylhexylmethoxycinnamat | 1,00 | - |
| Ethylhexyltriazon | 2,00 | - |
| Octocrylen | - | 1,00 |
| Bis-Ethylhexyloxyphenolmethoxyphenyltriazin | 3,00 | - |
| Magnolienrindenextrakt | 0,40 | 1,00 |
| Vitamin E Acetat | 0,10 | - |
| Vitamin C Phosphat | - | 0,20 |
| BHT | 0,10 | 0,05 |
| Parfüm, Konservierungsmittel | q.s. | q.s. |
| Natriumhydroxid | q.s. | q.s. |
| Wasser | ad 100,0 | ad 100,0 |

**Anti-Falten-Creme (Beispiel 12 und 13)**

| | **Nr. 12** | **Nr. 13** |
|---|---|---|
| Caprylsäure/Caprinsäuretriglyceride | 2,00 | 3,00 |
| Octyldodecanol | 2,50 | 2,50 |
| Cetearyl Alkohol | 3,00 | 3,00 |
| PEG-40 Stearat | 1,00 | 1,00 |
| Trinatrium EDTA + Wasser | 1,00 | 1,00 |
| Dimethicon | 0,35 | 0,35 |
| Cyclomethicon | 2,15 | 2,15 |
| C12-15 Alkylbenzoate | 2,00 | 2,50 |
| Glycerin | 5,00 | 7,00 |
| Xanthan Gum | 0,10 | 0,10 |
| Polyethylenglycol(20)sorbitanmonopalmitat | 2,50 | 3,00 |
| Natriumpolyacrylate | 0,30 | 0,30 |
| Natriummetabisulfit | - | 0,15 |
| Magnolienrindenextrakt | 0,15 | 0,15 |
| Phenoxyethanol | 0,30 | 0,40 |
| Methylparaben | 0,20 | 0,10 |
| Propylparaben | 0,05 | 0,10 |
| Wasser | ad 100 | ad 100 |

## Patentansprüche

1. Wirkstoffkombinationen aus
a) einem mit Hilfe von superkritischem CO₂ oder Ethanol oder einer Ethanol / Wasser Mischung gewonnenem Magnolienrindenextrakt mit einem Gehalt an Magnolol und/oder Honokiol
und
b) und einer oder mehreren ober- oder grenzflächenaktiven polyethoxylierten Verbindungen,
**dadurch gekennzeichnet, dass** die ober- oder grenzflächenaktiven polyethoxylierten Verbindung oder Verbindungen gewählt wird oder werden aus der Gruppe Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol-(30)stearat, Polyethylenglycol(40)stearat, Polyethylenglycol(100)stearat, Polyethylenglycol(200)stearat, Polyethylenglycol(400)stearat, Polyethylenglycol-(2)stearylether, Polyethylenglycol(21)stearylether, Polyethylenglycol-(20)cetylstearylether, Polyethylenglycol(40) hydriertes Rizinusöl, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

2. Verwendung
b) einer oder mehreren ober- oder grenzflächenaktiven polyethoxylierten Verbindungen zur Stabilisierung eines
a) mit Hilfe von superkritischem CO₂ oder Ethanol oder einer Ethanol / Wasser Mischung gewonnenen Magnolienrindenextraktes mit einem Gehalt an Magnolol und/oder Honokiol
gegen den Verderb durch Licht- und/oder Lufteinwirkung und/oder Temperatureinflüsse, **dadurch gekennzeichnet, dass** die ober- oder grenzflächenaktiven polyethoxylierten Verbindung oder Verbindungen gewählt wird oder werden aus der Gruppe Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol-(30)stearat, Polyethylenglycol(40)stearat, Polyethylenglycol(100)stearat, Polyethylenglycol(200)stearat, Polyethylenglycol(400)stearat, Polyethylenglycol-(2)stearylether, Polyethylenglycol(21)stearylether, Polyethylenglycol-(20)cetylstearylether, Polyethylenglycol(40) hydriertes Rizinusöl, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

3. Zubereitungen enthaltend Magnolienrindenextrakt gemäß Anspruch 1 mit einem Gehalt von 0,005 - 50,0 Gew.-%, insbesondere 0,01 - 20,0 Gew.-%, bevorzugt 0,02 - 10,0 Gew.-%, besonders bevorzugt 0,02 - 5,0 Gew, ganz besonders vorteilhaft 0,05 - 3,0 Gew.-%, jeweils bezogen auf das Gesamtgewicht der Zusammensetzung.

4. Verwendung nach Anspruch 2, bei der Wirkstoffkombinationen in kosmetischen oder dermatologischen Zubereitungen vorliegen, mit einem Gehalt von 0,005 - 50,0 Gew.-%, insbesondere 0,01 - 20,0 Gew.-%, bevorzugt 0,02 - 10,0 Gew.-%, besonders bevorzugt 0,02 - 5,0 Gew, ganz besonders vorteilhaft 0,05 - 3,0 Gew.-%, jeweils bezogen auf das Gesamtgewicht der Zusammensetzung.

5. Zubereitungen oder Verwendung nach einem der vorstehenden Ansprüche, wobei die Zubereitungen, 0,001 - 10 Gew.-% an einer oder mehreren ober- oder grenzflächenaktiven polyethoxylierten Verbindungen, bezogen auf das Gesamtgewicht der Zubereitungen enthalten.

6. Zubereitungen oder Verwendung nach einem der vorstehenden Ansprüche, wobei die ober- oder grenzflächenaktiven polyethoxylierten Verbindung oder Verbindungen gewählt wird oder werden aus der Gruppe Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol-(25)stearat, Polyethylenglycol(30)stearat, Polyethylenglycol(40)stearat, Polyethylenglycol(100)stearat, Polyethylenglycol(200)stearat, Polyethylenglycol(400)stearat, Polyethylenglycol(2)stearylether, Polyethylenglycol(21)stearylether, Polyethylenglycol(20)cetylstearylether, Polyethylenglycol(40) hydriertes Rizinusöl, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

7. Zubereitungen -oder Verwendung nach einem der vorstehenden Ansprüche, wobei als Magnolienrindenextrakt ein Extrakt aus der Rinde von Magnolia officinalis gewählt wird.

## Claims

1. Active ingredient combinations of
a) a magnolia bark extract having a content of magnolol and/or honokiol obtained with the aid of supercritical CO₂ or ethanol or an ethanol/water mixture
and
b) one or more surfactant or surface-active polyethoxylated compounds, **characterized in that** the surfactant or surface-active polyethoxylated compound or compounds is or are selected from the group comprising polyethylene glycol(8) stearate, polyethylene glycol(20) stearate, polyethylene glycol(21) stearate, polyethylene glycol(22) stearate, polyethylene glycol(23) stearate, polyethylene glycol(24) stearate, polyethylene glycol(25) stearate, polyethylene glycol(30) stearate, polyethylene glycol(40) stearate, polyethylene glycol(100) stearate, polyethylene glycol(200) stearate, polyethylene glycol(400) stearate, polyethylene glycol(2) stearyl ether, polyethylene glycol(21) stearyl ether, polyethylene glycol(20) cetyl/stearyl ether, polyethylene glycol(40) hydrogenated castor oil, polyethylene glycol(20) sorbitan monostearate, polyethylene glycol(20) sorbitan monoisostearate, polyethylene glycol(20) sorbitan monopalmitate, polyethylene glycol(20) sorbitan monooleate.

2. Use
b) of one or more surfactant or surface-active polyethoxylated compounds for stabilizing a
a) magnolia bark extract having a content of magnolol and/or honokiol obtained with the aid of supercritical CO₂ or ethanol or an ethanol/water mixture against deterioration by exposure to light and/or air and/or temperature influences, **characterized in that** the surfactant or surface-active polyethoxylated compound or compounds is or are selected from the group comprising polyethylene glycol(8) stearate, polyethylene glycol(20) stearate, polyethylene glycol(21) stearate, polyethylene glycol(22) stearate, polyethylene glycol(23) stearate, polyethylene glycol(24) stearate, polyethylene glycol(25) stearate, polyethylene glycol(30) stearate, polyethylene glycol(40) stearate, polyethylene glycol(100) stearate, polyethylene glycol(200) stearate, polyethylene glycol(400) stearate, polyethylene glycol(2) stearyl ether, polyethylene glycol(21) stearyl ether, polyethylene glycol(20) cetyl/stearyl ether, polyethylene glycol(40) hydrogenated castor oil, polyethylene glycol(20) sorbitan monostearate, polyethylene glycol(20) sorbitan monoisostearate, polyethylene glycol(20) sorbitan monopalmitate, polyethylene glycol(20) sorbitan monooleate.

3. Preparations comprising magnolia bark extract according to Claim 1 having a content of 0.005 - 50.0% by weight, in particular 0.01 - 20.0% by weight, preferably 0.02 - 10.0% by weight, particularly preferably 0.02 - 5.0% by weight, especially advantageously 0.05 - 3.0% by weight, based in each case on the total weight of the composition.

4. Use according to Claim 2, in which active ingredient combinations are present in cosmetic or dermatological preparations, having a content of 0.005 - 50.0% by weight, in particular 0.01 - 20.0% by weight, preferably 0.02 - 10.0% by weight, particularly preferably 0.02 - 5.0% by weight, especially advantageously 0.05 - 3.0% by weight, based in each case on the total weight of the composition.

5. Preparations or use according to any of the preceding claims, wherein the preparations comprise 0.001 - 10% by weight of one or more surfactant or surface-active polyethoxylated compounds, based on the total weight of the preparations.

6. Preparations or use according to any of the preceding claims, wherein the surfactant or surface-active polyethoxylated compound or compounds is or are selected from the group comprising polyethylene glycol(8) stearate, polyethylene glycol(20) stearate, polyethylene glycol(21) stearate, polyethylene glycol(22) stearate, polyethylene glycol(23) stearate, polyethylene glycol(24) stearate, polyethylene glycol(25) stearate, polyethylene glycol(30) stearate, polyethylene glycol(40) stearate, polyethylene glycol(100) stearate, polyethylene glycol(200) stearate, polyethylene glycol(400) stearate, polyethylene glycol(2) stearyl ether, polyethylene glycol(21) stearyl ether, polyethylene glycol(20) cetyl/stearyl ether, polyethylene glycol(40) hydrogenated castor oil, polyethylene glycol(20) sorbitan monostearate, polyethylene glycol(20) sorbitan monoisostearate, polyethylene glycol(20) sorbitan monopalmitate, polyethylene glycol(20) sorbitan monooleate.

7. Preparations or use according to any of the preceding claims, wherein the magnolia bark extract selected is an extract of the bark of Magnolia officinalis.

## Revendications

1. Combinaisons d'agents actifs, constituées par :
a) un extrait d'écorce de magnolia obtenu à l'aide de CO₂ supercritique ou d'éthanol ou d'un mélange éthanol/eau, ayant une teneur en magnolol et/ou honokiol,
et
b) un ou plusieurs composés polyéthoxylés tensioactifs,
**caractérisées en ce que** le composé ou les composés polyéthoxylés tensioactifs sont choisis dans le groupe constitué par le stéarate de polyéthylène glycol (8), le stéarate de polyéthylène glycol (20), le stéarate de polyéthylène glycol (21), le stéarate de polyéthylène glycol (22), le stéarate de polyéthylène glycol (23), le stéarate de polyéthylène glycol (24), le stéarate de polyéthylène glycol (25), le stéarate de polyéthylène glycol (30), le stéarate de polyéthylène glycol (40), le stéarate de polyéthylène glycol (100), le stéarate de polyéthylène glycol (200), le stéarate de polyéthylène glycol (400), l'éther stéarylique de polyéthylène glycol (2), l'éther stéarylique de polyéthylène glycol (21), l'éther cétylstéarylique de polyéthylène glycol (20), l'huile de ricin hydrogénée de polyéthylène glycol (40), le monostéarate de sorbitane de polyéthylène glycol (20), le monoisostéarate de sorbitane de polyéthylène glycol (20), le monopalmitate de sorbitane de polyéthylène glycol (20), le monooléate de sorbitane de polyéthylène glycol (20).

2. Utilisation de
b) un ou plusieurs composés polyéthoxylés tensioactifs pour la stabilisation de
a) un extrait d'écorce de magnolia obtenu à l'aide de CO₂ supercritique ou d'éthanol ou d'un mélange éthanol/eau, ayant une teneur en magnolol et/ou honokiol, contre la détérioration par l'action de la lumière et/ou de l'air et/ou les effets de la température, **caractérisée en ce que** le composé ou les composés polyéthoxylés tensioactifs sont choisis dans le groupe constitué par le stéarate de polyéthylène glycol (8), le stéarate de polyéthylène glycol (20), le stéarate de polyéthylène glycol (21), le stéarate de polyéthylène glycol (22), le stéarate de polyéthylène glycol (23), le stéarate de polyéthylène glycol (24), le stéarate de polyéthylène glycol (25), le stéarate de polyéthylène glycol (30), le stéarate de polyéthylène glycol (40), le stéarate de polyéthylène glycol (100), le stéarate de polyéthylène glycol (200), le stéarate de polyéthylène glycol (400), l'éther stéarylique de polyéthylène glycol (2), l'éther stéarylique de polyéthylène glycol (21), l'éther cétylstéarylique de polyéthylène glycol (20), l'huile de ricin hydrogénée de polyéthylène glycol (40), le monostéarate de sorbitane de polyéthylène glycol (20), le monoisostéarate de sorbitane de polyéthylène glycol (20), le monopalmitate de sorbitane de polyéthylène glycol (20), le monooléate de sorbitane de polyéthylène glycol (20).

3. Préparations contenant un extrait d'écorce de magnolia selon la revendication 1, ayant une teneur de 0,005 à 50,0 % en poids, notamment de 0,01 à 20,0 % en poids, de préférence de 0,02 à 10,0 % en poids, de manière particulièrement préférée de 0,02 à 5,0 % en poids, de manière tout particulièrement préférée de 0,05 à 3,0 % en poids, à chaque fois par rapport au poids total de la composition.

4. Utilisation selon la revendication 2, dans laquelle les combinaisons d'agents actifs se trouvent dans des préparations cosmétiques ou dermatologiques, en une teneur de 0,005 à 50,0 % en poids, notamment de 0,01 à 20,0 % en poids, de préférence de 0,02 à 10,0 % en poids, de manière particulièrement préférée de 0,02 à 5,0 % en poids, de manière tout particulièrement avantageuse de 0,05 à 3,0 % en poids, à chaque fois par rapport au poids total de la composition.

5. Préparations ou utilisation selon l'une quelconque des revendications précédentes, dans lesquelles les préparations contiennent 0,001 à 10 % en poids d'un ou de plusieurs composés polyéthoxylés tensioactifs, par rapport au poids total des préparations.

6. Préparations ou utilisation selon l'une quelconque des revendications précédentes, dans lesquelles le composé ou les composés polyéthoxylés tensioactifs sont choisis dans le groupe constitué par le stéarate de polyéthylène glycol (8), le stéarate de polyéthylène glycol (20), le stéarate de polyéthylène glycol (21), le stéarate de polyéthylène glycol (22), le stéarate de polyéthylène glycol (23), le stéarate de polyéthylène glycol (24), le stéarate de polyéthylène glycol (25), le stéarate de polyéthylène glycol (30), le stéarate de polyéthylène glycol (40), le stéarate de polyéthylène glycol (100), le stéarate de polyéthylène glycol (200), le stéarate de polyéthylène glycol (400), l'éther stéarylique de polyéthylène glycol (2), l'éther stéarylique de polyéthylène glycol (21), l'éther cétylstéarylique de polyéthylène glycol (20), l'huile de ricin hydrogénée de polyéthylène glycol (40), le monostéarate de sorbitane de polyéthylène glycol (20), le monoisostéarate de sorbitane de polyéthylène glycol (20), le monopalmitate de sorbitane de polyéthylène glycol (20), le monooléate de sorbitane de polyéthylène glycol (20).

7. Préparations ou utilisation selon l'une quelconque des revendications précédentes, dans lesquelles un extrait de l'écorce de magnolia officinalis est choisi en tant qu'extrait d'écorce de magnolia.
